# EUROPEAN PATENT APPLICATION

(11) **EP 4 454 583 A1**
(43) Date of publication of application: **30.10.2024**
(21) Application number: 22910863.4
(22) Date of filing: 06.12.2022
(51) Int. Cl.: A61B 18/14

(54) **PLASMA GUIDE WIRE**

(30) Priority: 22.12.2021 JP 2021208108
(71) Applicant: ASAHI INTECC CO., LTD., Seto-shi, Aichi, 489-0071 (JP)
(72) Inventor: IWATA, Naozumi, Seto-shi, Aichi 489-0071 (JP); ICHIKAWA, Tomoki, Seto-shi, Aichi 489-0071 (JP); OKAMOTO, Shoma, Seto-shi, Aichi 489-0071 (JP)
(74) Representative: Winter, Brandl - Partnerschaft mbB
(86) International application number: PCT/JP2022/044830
(87) International publication number: WO 2023/120149

(57) **Abstract**

A plasma guide wire includes a guide wire main body, a first tube that is made of an insulating resin and covers a distal end side of the guide wire main body, a second tube that is made of an insulating resin and covers a proximal end side of the guide wire main body, and a third tube that is made of an insulating resin, covers an intermediate portion of the guide wire main body located between the distal end side and the proximal end side, has a distal end portion joined to a proximal end portion of the first tube, and has a proximal end portion joined to a distal end portion of the second tube. At least any one of the first tube, the second tube, and the third tube forms a gas layer filled with gas, in conjunction with the guide wire main body.

## Description

### TECHNICAL FIELD

The disclosed embodiments relate to a plasma guide wire.

### BACKGROUND ART

In recent years, a plasma ablation treatment for ablating (cauterizing) a biological tissue by using a plasma flow has been known as a treatment method for arrhythmia that causes an abnormality in the beating rhythm of the heart or Chronic Total Occlusion (CTO: Chronic Total Occlusion) in which the inside of a blood vessel is occluded by a lesion. For example, Patent Literature 1 discloses a drainage tube indwelling device for an endoscope which may be used in such a plasma ablation treatment. The drainage tube indwelling device for an endoscope described in Patent Literature 1 includes a drainage tube including an electrically insulating flexible tube, a guide wire including a conductive flexible wire, and a high-frequency power supply connection means for applying a high-frequency current to the guide wire.

### CITATION LIST

### Patent Literature

Patent Literature 1: JP 2000-084065 A

### SUMMARY OF INVENTION

### TECHNICAL PROBLEM

However, in the drainage tube indwelling device for an endoscope described in Patent Literature 1, since the drainage tube for providing electrical insulation directly covers the outer peripheral surface of the guide wire, there is a problem in that insulation performance is poor. In particular, in the plasma ablation treatment, a high voltage of about 700 V is applied to the guide wire, which is different from the conventional thermal ablation treatment, and therefore, there is a need to further improve the insulation performance. In addition, in the drainage tube indwelling device for an endoscope described in Patent Literature 1, since the guide wire to which a high-frequency current is applied may be inserted into and removed from the drainage tube for providing electrical insulation, there is a problem in that the guide wire needs to be handled with care. Such a problem is not limited to the vascular system, but is common to all plasma guide wires which are inserted into body lumens such as a lymphatic system, a biliary system, a urinary system, an airway system, a digestive system, a secretory gland, and a reproductive organ for plasma ablation treatment. In addition, the plasma guide wire is required to be improved in flexibility, operability, and the like.

The disclosed embodiments have been made to solve at least a part of the above-described issues and have an object to improve the insulation performance of a plasma guide wire.

### SOLUTION TO PROBLEM

The disclosed embodiments have been made to solve at least a part of the above-described issues and may be implemented as the following aspects.

(1) According to one aspect of the disclosed embodiments, a plasma guide wire is provided. The plasma guide wire includes a guide wire main body including a core shaft and a coil body provided to surround a portion of the core shaft on a distal end side, a hollow cylindrical first tube that is made of an insulating resin and covers a distal end side of the guide wire main body, a hollow cylindrical second tube that is made of an insulating resin and covers a proximal end side of the guide wire main body, and a hollow cylindrical third tube that is made of an insulating resin, covers an intermediate portion of the guide wire main body located between the distal end side and the proximal end side, has a distal end portion joined to a proximal end portion of the first tube, and has a proximal end portion joined to a distal end portion of the second tube, and at least any one of the first tube, the second tube, and the third tube forms a gas layer filled with gas, in conjunction with the guide wire main body.

With this configuration, since the guide wire main body is covered with the first tube, the second tube, and the third tube each made of an insulating resin, it is possible to insulate the guide wire main body from the outside and to give insulating performance to the plasma guide wire. In addition, the gas layer filled with gas is formed between at least any one of the first tube, the second tube, and the third tube and the guide wire main body. Here, when air is given as an example of the gas, the volume resistivity of air is higher than the volume resistivity of resin, and thus it may be said that air has a higher insulation performance than resin. In addition, it is known that, for example, sulfur hexafluoride (SF6) gas has better insulating properties than air. Therefore, by providing the plasma guide wire with the gas layer filled with the gas having high insulation performance described above, the insulation performance of the plasma guide wire may be improved as compared with a configuration without the gas layer. As a result, the operator may safely perform the plasma ablation treatment using the plasma guide wire.

(2) In the plasma guide wire according to the above-described aspect, the gas layer filled with the gas may be formed between the first tube and the guide wire main body.

The first tube covering the distal end side of the guide wire main body may be formed to be thinner or may be made of a flexible resin material in order to improve flexibility as compared with the second tube covering the proximal end side of the guide wire main body or the third tube covering the intermediate portion of the guide wire main body. In this regard, with this configuration, since the gas layer filled with gas is formed between the first tube and the guide wire main body, even when the insulation performance of the first tube itself is inferior to that of the second tube or the third tube, the gas layer may improve the insulation performance of the portion where the first tube is located (in other words, the distal end side portion of the plasma guide wire).

(3) In the plasma guide wire according to the above-described aspect, a thickness of the gas layer may be 1 µm or more and 100 µm or less.

With this configuration, since the thickness of the gas layer is 1 µm or more, it is possible to secure an insulation distance necessary for suppressing electric leakage, and it is possible to suppress the occurrence of electric leakage and the occurrence of dielectric breakdown due to electric leakage. Further, even when electric leakage occurs, the amount of leakage current may be reduced. In addition, since the thickness of the gas layer is 100 µm or less, both the insulation performance and the usability (for example, delivery performance and operability) of the plasma guide wire may be achieved.

(4) The plasma guide wire according to the above-described aspect may further include a fixation portion that fixes any of the first tube, the second tube, and the third tube to the guide wire main body, and the fixation portion may not be provided in at least any one of a first section in which the guide wire main body is covered with the first tube, a second section in which the guide wire main body is covered with the second tube, and a third section in which the guide wire main body is covered with the third tube.

With this configuration, since no fixation portion is provided in at least any one of the first section, the second section, and the third section, the flexibility of the plasma guide wire may be improved as compared with the configuration in which the fixation portion is provided in all of the first, second, and third sections.

(5) The plasma guide wire according to the above-described aspect may further include a distal end fixation portion that is provided at a distal end portion of the first tube and fixes the first tube and the guide wire main body, and a proximal end fixation portion that is provided at a proximal end portion of the second tube and fixes the second tube and the guide wire main body, and the distal end fixation portion and the proximal end fixation portion may block flow of the gas between inside and outside of the plasma guide wire.

With this configuration, the distal end fixation portion is provided at the distal end portion of the first tube, the proximal end fixation portion is provided at the proximal end portion of the second tube, and the distal end fixation portion and the proximal end fixation portion block the flow of gas between the inside and the outside of the plasma guide wire. Therefore, it is possible to suppress the gas forming the gas layer inside the plasma guide wire from leaking to the outside of the plasma guide wire when the plasma guide wire is gripped or curved. As a result, the insulation performance of the plasma guide wire may be further improved.

(6) In the plasma guide wire according to the above-described aspect, an outer diameter of the intermediate portion of the guide wire main body may be smaller than an outer diameter of the guide wire main body on the distal end side and may be smaller than an outer diameter of the guide wire main body on the proximal end side, and an outer diameter of the third tube covering the intermediate portion may be smaller than an outer diameter of the first tube covering the distal end side and may be smaller than an outer diameter of the second tube covering the proximal end side.

With this configuration, the outer shape of the plasma guide wire (specifically, the outer shapes of the first, second, and third tubes) may be formed along the outer shape of the guide wire main body. As a result, the diameter of the plasma guide wire may be reduced, and the mechanical performance (for example, torquability and support characteristics) of the plasma guide wire may be maintained.

(7) In the plasma guide wire according to the above-described aspect, the third tube may be provided such that the distal end portion of the third tube is overlapped with the proximal end portion of the first tube and the proximal end portion of the third tube is overlapped with the distal end portion of the second tube, an outer peripheral surface of the distal end portion of the third tube may be joined to an inner peripheral surface of the proximal end portion of the first tube, and an outer peripheral surface of the proximal end portion of the third tube may be joined to an inner peripheral surface of the distal end portion of the second tube.

With this configuration, the third tube is provided such that the distal end portion of the third tube is overlapped with the proximal end portion of the first tube and the proximal end portion of the third tube is overlapped with the distal end portion of the second tube, and the outer peripheral surface of the third tube is joined to the inner peripheral surfaces of the other tubes (the first and second tubes) so that leakage of gas from the boundary between the third tube and the other tubes (the first and second tubes) may be suppressed.

(8) In the plasma guide wire according to the above-described aspect, an intermediate portion of the third tube located at least between the distal end portion and the proximal end portion may not be covered with the first tube and the second tube and may be exposed to outside.

With this configuration, since the intermediate portion of the third tube located at least between the distal end portion and the proximal end portion is not covered with the first tube and the second tube and is exposed to the outside, the diameter of the plasma guide wire in the third section (the section in which the guide wire main body is covered with the third tube) may be reduced, and flexible configuration is possible.

Furthermore, the disclosed embodiments may be realized in various modes and may be realized in the form of, for example, a plasma guide wire, a plasma ablation system including a plasma guide wire and an RF generator, a guide wire that ablates (cauterizes) biological tissue using heat instead of plasma, and a method for manufacturing a plasma guide wire or a guide wire.

### BRIEF DESCRIPTION OF DRAWINGS

Fig. 1 is an explanatory view exemplifying a sectional configuration of a plasma guide wire.
Figs. 2A and 2B are diagrams illustrating a guide wire main body and first, second, and third tubes extracted from Fig. 1.
Fig. 3 is an explanatory view of a gas layer.
Fig. 4 is an explanatory view exemplifying a transverse sectional configuration taken along the line A-A of Fig. 3.
Figs. 5A and 5B are enlarged views of a portion of Fig. 3.
Fig. 6 is an explanatory view exemplifying a sectional configuration of a plasma guide wire according to a second embodiment.
Fig. 7 is an explanatory view exemplifying a sectional configuration of a plasma guide wire according to a third embodiment.
Fig. 8 is an explanatory view exemplifying a sectional configuration of a plasma guide wire according to a fourth embodiment.
Fig. 9 is an explanatory view exemplifying a sectional configuration of a plasma guide wire according to a fifth embodiment.
Fig. 10 is an explanatory view exemplifying a sectional configuration of a plasma guide wire according to a sixth embodiment.
Fig. 11 is an explanatory view exemplifying a sectional configuration of a plasma guide wire according to a seventh embodiment.

### EMBODIMENTS OF THE INVENTION

### <First Embodiment>

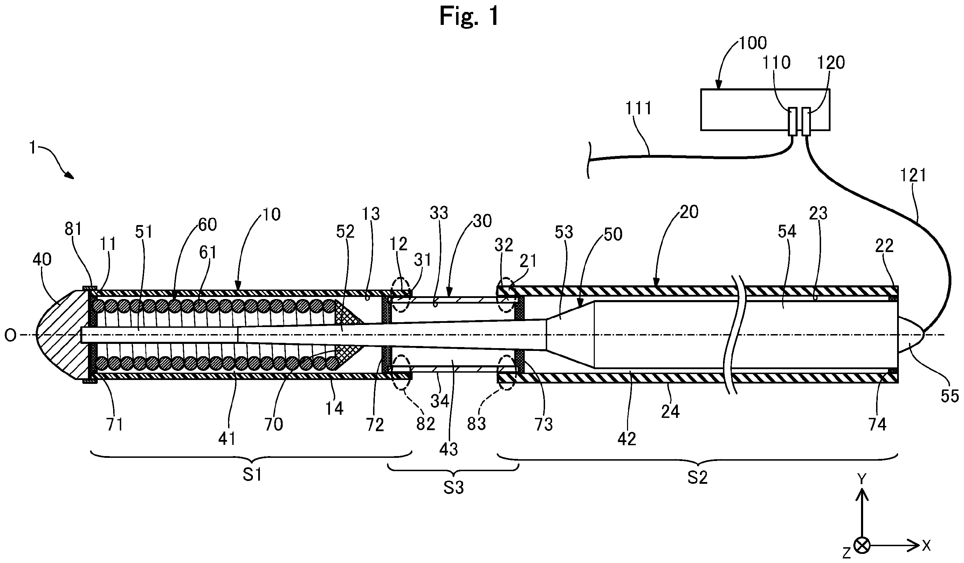

Fig. 1 is an explanatory view exemplifying a sectional configuration of a plasma guide wire 1. The plasma guide wire 1 is a device used for the purpose of Chronic Total Occlusion (CTO: Chronic Total Occlusion) recanalization or treatment for mild to moderate stenosis, significant stenosis, arrhythmia, or the like, by ablating (cauterizing) a biological tissue using a plasma flow. Hereinafter, a case where the plasma guide wire 1 is used for CTO recanalization in blood vessels will be described as an example; however, the plasma guide wire 1 may be used by being inserted into body lumens such as a lymphatic system, a biliary system, a urinary system, an airway system, a digestive system, a secretory gland, and a reproductive organ as well as a vascular system.

In Fig. 1, the axis passing through the center of the plasma guide wire 1 is represented by an axis line O (a dashed-dotted line). In the example of Fig. 1, the axis line O coincides with the axis passing through the center of each constituent member of the plasma guide wire 1, that is, a first tube 10, a second tube 20, a third tube 30, a distal end electrode 40, a core shaft 50, and a coil body 60. However, the axis line O may be different from the center axis of each constituent member of the plasma guide wire 1. Furthermore, Fig. 1 illustrates XYZ axes orthogonal to each other. The X-axis corresponds to the longitudinal direction of the plasma guide wire 1, the Y-axis corresponds to the height direction of the plasma guide wire 1, and the Z-axis corresponds to the width direction of the plasma guide wire 1. The left side (-X axis direction) of Fig. 1 is referred to as the "distal end side" of the plasma guide wire 1 and each constituent member, and the right side (+X axis direction) of Fig. 1 is referred to as the "proximal end side" of the plasma guide wire 1 and each constituent member. Of both ends in the longitudinal direction (the X-axis direction), one end located on the distal end side is referred to as the "distal end", and the other end located on the proximal end side is referred to as the "proximal end". The distal end and its vicinity are referred to as the "distal end portion", and the proximal end and its vicinity are referred to as the "proximal end portion". The distal end side is inserted into a living body, and the proximal end side is operated by an operator such as a doctor. These points are common to Fig. 1 and the subsequent figures.

The plasma guide wire 1 has an elongated outer shape and includes the first tube 10, the second tube 20, the third tube 30, the core shaft 50, the distal end electrode 40, the coil body 60, a coil fixation portion 70, a first fixation portion 71, a second fixation portion 72, a third fixation portion 73, a fourth fixation portion 74, and a distal end marker 81.

The distal end electrode 40 is a member that has conductivity and causes discharge between the distal end electrode 40 and another electrode not illustrated. The distal end electrode 40 is provided on the most distal end side of the plasma guide wire 1 (in other words, the distal end portion of the plasma guide wire 1). The distal end electrode 40 has an outer shape whose diameter is reduced from the proximal end side to the distal end side in order to smoothly advance the plasma guide wire 1 in a blood vessel. A proximal end portion of the distal end electrode 40 is joined to a distal end portion 11 of the first tube 10 and a distal end portion of the core shaft 50. Any joining agent such as an epoxy-based adhesive may be used for joining. Further, laser welding or the like may be used as a joining means. The other electrodes are provided in another device not illustrated. Any configuration may be adopted as another device. For example, another device may be a catheter in which another electrode is provided at the distal end portion and the plasma guide wire 1 is inserted into the inside, may be another guide wire in which another electrode is provided at the distal end portion, or may be a pad including another electrode.

The core shaft 50 is a member that has conductivity and forms the center axis of the plasma guide wire 1. The core shaft 50 has an elongated outer shape extending in the longitudinal direction of the plasma guide wire 1. The core shaft 50 includes a small diameter portion 51, a first tapered portion 52, a second tapered portion 53, and a large diameter portion 54 from the distal end toward the proximal end. The small diameter portion 51 is a portion where the outer diameter of the core shaft 50 is the thinnest and has a substantially columnar shape having a substantially constant outer diameter from the distal end to the proximal end. The first tapered portion 52 is a portion provided between the small diameter portion 51 and the second tapered portion 53 and has an outer shape whose diameter is reduced from the proximal end side to the distal end side. The second tapered portion 53 is a portion provided between the first tapered portion 52 and the large diameter portion 54 and has an outer shape in which the outer diameter is reduced from the proximal end side to the distal end side at an angle different from that of the first tapered portion 52. The large diameter portion 54 is a portion where the outer diameter of the core shaft 50 is the thickest and has a substantially columnar shape having a substantially constant outer diameter from the distal end to the proximal end. The proximal end portion 55 of the large diameter portion 54 is a portion where a proximal end surface of the large diameter portion 54 bulges.

According to the present embodiment, the term "substantially constant" has the same meaning as the term "approximately constant" and means being approximately constant while allowing deviations due to manufacturing errors, or the like. In addition, according to the present embodiment, the "outer diameter" and the "inner diameter" adopt the length of the longest portion in an arbitrary transverse section when the transverse section of the member (or the inner cavity) is elliptical.

An RF generator 100 illustrated in Fig. 1 is a device that outputs high-frequency power between a first terminal 110 and a second terminal 120. A second cable 121 is connected to the proximal end portion 55 of the core shaft 50 of the plasma guide wire 1. The second cable 121 is an electric wire having conductivity. The second cable 121 extends from the second terminal 120 of the RF generator 100 and electrically connects the RF generator 100 and the plasma guide wire 1. In addition, a first cable 111 is connected to another device having another electrode described above. The first cable 111 is an electric wire having conductivity. The first cable 111 extends from the first terminal 110 of the RF generator 100 and electrically connects the RF generator 100 to another device. Further, the first cable 111 and the second cable 121 may be provided with a cable connector (a connection terminal to physically and electrically connect cables).

The coil body 60 has conductivity and is provided to surround a portion of the core shaft 50 on the distal end side. In the example of Fig. 1, the coil body 60 is provided to surround, in the core shaft 50, the small diameter portion 51 and a portion of the first tapered portion 52 on the distal end side. The coil body 60 is formed by spirally winding a wire 61 having conductivity. The coil body 60 may be a single-thread coil formed by winding one wire as a single thread, a multi-thread coil formed by winding a plurality of wires as a multi-thread, a single-thread strand coil formed by winding a strand, which is formed by twisting a plurality of wires, as a single thread, or a multi-thread strand coil using a plurality of strands, which is formed by twisting a plurality of wires, and winding each strand as a multi-thread. Further, the core shaft 50 and the coil body 60 are also collectively referred to as the "guide wire main body".

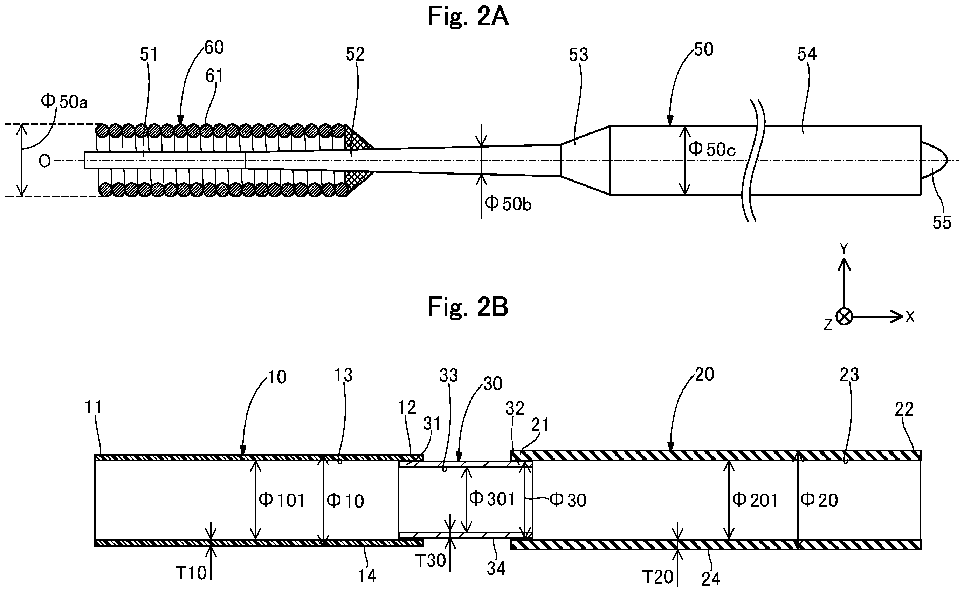

Figs. 2A and 2B are diagrams illustrating the guide wire main body and the first, second, and third tubes 10, 20, 30 extracted from Fig. 1. Fig. 2A is a diagram of the guide wire main body extracted, and Fig. 2B is a diagram of the first, second, and third tubes 10, 20, 30 extracted. Hereinafter, a portion of the guide wire main body on which the coil body 60 is provided is also referred to as the "distal end side of the guide wire main body". In addition, a portion of the guide wire main body where the second tapered portion 53 and the large diameter portion 54 are provided is also referred to as the "proximal end side of the guide wire main body". Further, a portion of the guide wire main body located between the distal end side and the proximal end side (specifically, a portion where the first tapered portion 52 is exposed from the coil body 60) is also referred to as the "intermediate portion of the guide wire main body". Fig. 2A illustrates outer diameters φ50a, φ50b, φ50c for the distal end side, the intermediate portion, and the proximal end side of the guide wire main body, respectively. The outer diameter φ50a is equal to the outer diameter of the coil body 60. The outer diameter φ50b is equal to the outer diameter of an arbitrary portion in the portion where the first tapered portion 52 is exposed from the coil body 60. The outer diameter φ50c is equal to the outer diameter of the large diameter portion 54. As illustrated in Fig. 2A, the outer diameter φ50b of the intermediate portion of the guide wire main body is smaller than the outer diameter φ50a on the distal end side and is smaller than the outer diameter φ50c on the proximal end side.

The first tube 10 is a hollow cylindrical tubular body made of an insulating resin. The first tube 10 is provided closer to the proximal end side than the distal end electrode 40 and covers the distal end side of the guide wire main body. In the example of Fig. 1, the first tube 10 covers, in the guide wire main body, the outer periphery of the coil body 60 and a portion of the first tapered portion 52 of the core shaft 50 on the distal end side, exposed from the coil body 60 on the proximal end side of the coil body 60. As illustrated in Figs. 2A and 2B, an inner diameter φ 101 of the first tube 10 is larger than the outer diameter φ50a on the distal end side of the guide wire main body. A thickness T10 of the first tube 10 may be arbitrarily determined.

The second tube 20 is a hollow cylindrical tubular body made of an insulating resin. The second tube 20 is provided closer to the proximal end side than the third tube 30 and covers the proximal end side of the guide wire main body. In the example of Fig. 1, the second tube 20 covers, in the guide wire main body, the proximal end portion of the first tapered portion 52, the second tapered portion 53, and the large diameter portion 54 of the core shaft 50. Further, the proximal end portion 55 of the large diameter portion 54 is not covered with the second tube 20 and is exposed to the outside. As illustrated in Figs. 2A and 2B, an inner diameter φ201 of the second tube 20 is larger than the outer diameter φ50c on the proximal end side of the guide wire main body. A thickness T20 of the second tube 20 may be arbitrarily determined.

The third tube 30 is a hollow cylindrical tubular body made of an insulating resin. The third tube 30 is provided between the first tube 10 and the second tube 20 and covers the intermediate portion of the guide wire main body. In other words, the third tube 30 covers a portion located in the middle of the guide wire main body that is covered with neither the first tube 10 nor the second tube 20. In the example of Fig. 1, the third tube 30 covers a portion of the first tapered portion 52 of the core shaft 50 in the guide wire main body. As illustrated in Figs. 2A and 2B, an inner diameter φ301 of the third tube 30 is larger than the outer diameter φ50b of the intermediate portion of the guide wire main body. A thickness T30 of the third tube 30 may be arbitrarily determined. The thicknesses T10, T20, T30 may be the same or different from each other.

As illustrated in Fig. 2B, a distal end portion 31 of the third tube 30 is joined to a proximal end portion 12 of the first tube 10. Further, a proximal end portion 32 of the third tube 30 is joined to a distal end portion 21 of the second tube 20. Here, an outer diameter φ30 of the third tube 30 is smaller than an outer diameter φ10 of the first tube 10 and is smaller than an outer diameter φ20 of the second tube 20. Further, as illustrated in Fig. 2B, the third tube 30 is provided such that the distal end portion 31 of the third tube 30 is overlapped with the proximal end portion 12 of the first tube 10 and the proximal end portion 32 of the third tube 30 is overlapped with the distal end portion 21 of the second tube 20. Therefore, an outer peripheral surface 34 of the distal end portion 31 of the third tube 30 is joined to an inner peripheral surface 13 of the proximal end portion 12 of the first tube 10. Similarly, an outer peripheral surface 34 of the proximal end portion 32 of the third tube 30 is joined to an inner peripheral surface 23 of the distal end portion 21 of the second tube 20. A portion of the third tube 30 located between the distal end portion 31 and the proximal end portion 32 (also referred to as the "intermediate portion" of the third tube 30) is not covered with the first tube 10 or the second tube 20 and is exposed to the outside.

The first tube 10, the second tube 20, and the third tube 30 may be bonded by using any bonding agent such as an epoxy-based adhesive. In Fig. 1, a joint portion between the third tube 30 and the first tube 10 is illustrated as a distal end side joint portion 82 (a circle frame by a broken line), and a joint portion between the third tube 30 and the second tube 20 is illustrated as a proximal end side joint portion 83 (a circle frame by a broken line). As illustrated in Fig. 2B, the joined body of the first, second, and third tubes 10, 20, 30 according to the present embodiment has a constricted shape at the intermediate portion where the third tube 30 is provided.

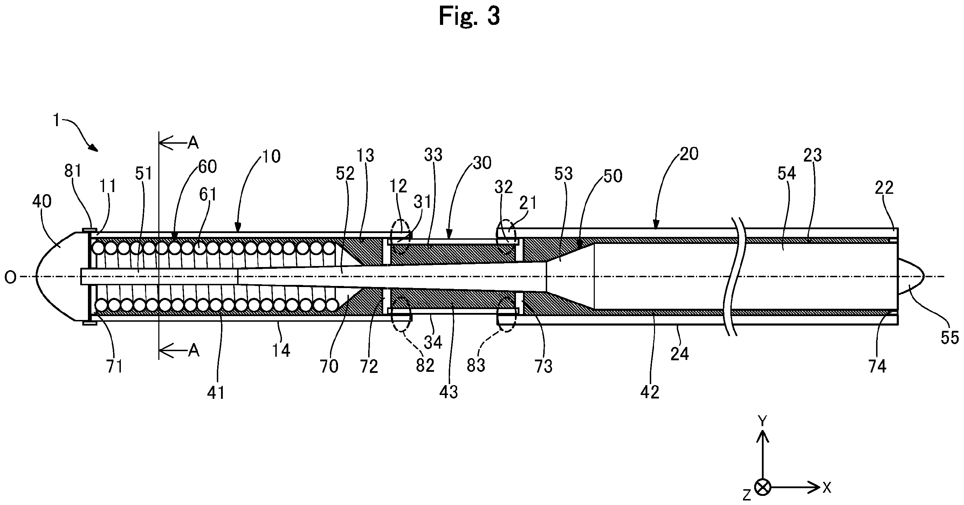

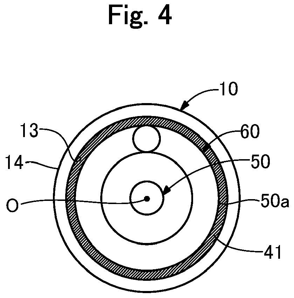

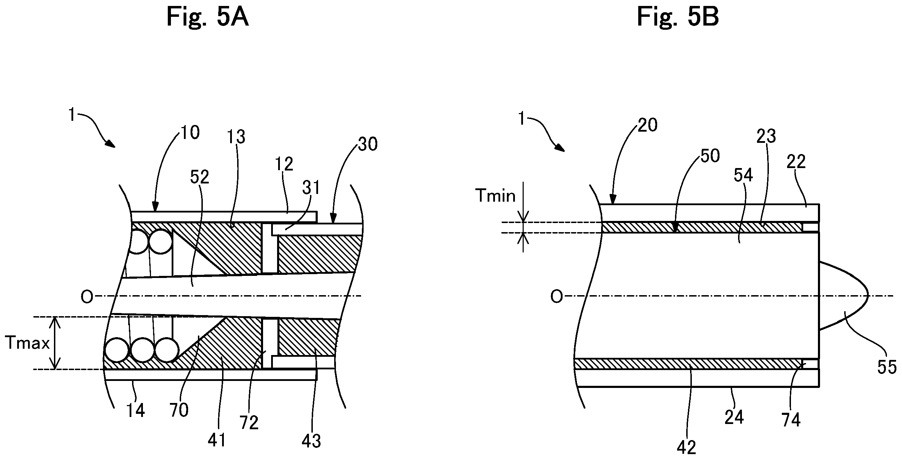

Fig. 3 is an explanatory view of gas layers 41, 42, 43. Fig. 4 is an explanatory view exemplifying a transverse sectional configuration taken along the line A-A of Fig. 3. Figs. 5A and 5B are enlarged views of a portion of Fig. 3. Fig. 5A is an enlarged view illustrating the vicinity of the proximal end portion of the coil body 60 of the plasma guide wire 1. Fig. 5B is an enlarged view illustrating the vicinity of the proximal end portion of the core shaft 50 in the plasma guide wire 1. In Figs. 3 to 5B, for convenience of explanation, only the gas layers 41, 42, 43 of the plasma guide wire 1 illustrated in Fig. 1 are hatched with oblique lines.

As illustrated in Figs. 3 to 5B, the gas layer 41 filled with gas is formed between the first tube 10 and the guide wire main body. Specifically, in the first tube 10, the gas layer 41 is formed between the inner peripheral surface 13 of the first tube 10 and the outer peripheral surface of the guide wire main body (specifically, the outer peripheral surface of the coil body 60 and the outer peripheral surface of the first tapered portion 52 exposed from the coil body 60). As illustrated in Fig. 4, the gas layer 41 is provided entirely in the circumferential direction between the first tube 10 and the guide wire main body. In addition, the gas layer 41 is provided entirely in the longitudinal direction from the distal end portion to the proximal end portion of the first tube 10 (specifically, entirely in the longitudinal direction from the proximal end of the first fixation portion 71 to the distal end of the second fixation portion 72) except for the portions where the first fixation portion 71 and the second fixation portion 72 are provided.

As illustrated in Figs. 3 to 5B, the gas layer 42 filled with gas is formed between the second tube 20 and the guide wire main body. Specifically, in the second tube 20, the gas layer 42 is formed between the inner peripheral surface 23 of the second tube 20 and the outer peripheral surface of the guide wire main body (specifically, the outer peripheral surface of the proximal end portion of the first tapered portion 52, the outer peripheral surface of the second tapered portion 53, and the outer peripheral surface of the large diameter portion 54). Similarly to the gas layer 41, the gas layer 42 is provided entirely in the circumferential direction between the second tube 20 and the guide wire main body. In addition, the gas layer 42 is provided entirely in the longitudinal direction from the distal end portion to the proximal end portion of the second tube 20 (specifically, entirely in the longitudinal direction from the proximal end of the third fixation portion 73 to the distal end of the fourth fixation portion 74) except for the portions where the third fixation portion 73 and the fourth fixation portion 74 are provided.

As illustrated in Figs. 3 to 5B, the gas layer 43 filled with gas is formed between the third tube 30 and the guide wire main body. Specifically, in the third tube 30, the gas layer 43 is formed between an inner peripheral surface 33 of the third tube 30 and the outer peripheral surface of the guide wire main body (specifically, the outer peripheral surface of the first tapered portion 52 exposed from the coil body 60). Similarly to the gas layer 41, the gas layer 43 is provided entirely in the circumferential direction provided between the third tube 30 and the guide wire main body. In addition, the gas layer 43 is provided entirely in the longitudinal direction from the distal end portion to the proximal end portion of the third tube 30 (specifically, entirely in the longitudinal direction from the proximal end of the second fixation portion 72 to the distal end of the third fixation portion 73) except for the portions where the second fixation portion 72 and the third fixation portion 73 are provided.

As the gas forming the gas layers 41, 42, 43, any gas may be used as long as the gas has higher insulation performance than the insulating resin forming the first, second, and third tubes 10, 20, 30. For example, air, sulfur hexafluoride (SF6) gas, or hydrogen (H₂) gas may be used as the gas forming the gas layers 41, 42, 43. When air is used as the gas, the gas layers 41, 42, 43 may also be referred to as the air layers 41, 42, 43.

Here, the thickness of the gas layer 41 is defined as the distance between the inner peripheral surface 13 of the first tube 10 and the guide wire main body. Similarly, the thickness of the gas layer 42 is defined as the distance between the inner peripheral surface 23 of the second tube 20 and the guide wire main body, and the thickness of the gas layer 43 is defined as the distance between the inner peripheral surface 33 of the third tube 30 and the guide wire main body. In the plasma guide wire 1 according to the present embodiment, the thickness of each of the gas layers 41, 42, 43 is 1 µm or more and 100 µm or less. In the plasma guide wire 1 according to the present embodiment, a maximum value Tmax of the thicknesses of the gas layers 41, 42, 43 is the distance of the portion illustrated in Fig. 5A, that is, the distance between the outer peripheral surface of the first tapered portion 52 and the inner peripheral surface 13 of the first tube 10 at the position corresponding to the proximal end of the coil fixation portion 70 that fixes the proximal end portion of the coil body 60 and the core shaft 50. Further, in the plasma guide wire 1 according to the present embodiment, a minimum value Tmin of the thicknesses of the gas layers 41, 42, 43 is the distance of the portion illustrated in Fig. 5B, that is, the distance between the outer peripheral surface of the large diameter portion 54 and the inner peripheral surface 23 of the second tube 20.

Returning to Fig. 1, the description will be continued. The coil fixation portion 70 is a member that fixes the proximal end portion of the coil body 60 and a portion of the first tapered portion 52 of the core shaft 50. The first fixation portion 71 is a member that is provided at the distal end portion 11 of the first tube 10 and fixes the distal end portion 11 of the first tube 10 and the guide wire main body (specifically, the distal end portion of the coil body 60 and the distal end portion of the small diameter portion 51). As illustrated in Fig. 1, the first fixation portion 71 is provided entirely in the circumferential direction between the inner peripheral surface 13 of the first tube 10 and the outer peripheral surface of the small diameter portion 51 and blocks the flow of gas between the inside and outside of the plasma guide wire 1 (specifically, the flow of the gas forming the gas layer 41). Further, the first fixation portion 71 corresponds to a "distal end fixation portion".

The second fixation portion 72 is a member that is provided at the distal end portion 31 of the third tube 30 and fixes the distal end portion 31 of the third tube 30, the proximal end portion 12 of the first tube 10, and the guide wire main body (specifically, a portion of the first tapered portion 52). As illustrated in Fig. 1, the second fixation portion 72 is provided entirely in the circumferential direction between the inner peripheral surface 13 of the first tube 10 and the inner peripheral surface 33 of the third tube 30 and the outer peripheral surface of the first tapered portion 52 and blocks the flow of gas between the gas layer 41 and the gas layer 43.

The third fixation portion 73 is a member that is provided at the proximal end portion 32 of the third tube 30 and fixes the proximal end portion 32 of the third tube 30, the distal end portion 21 of the second tube 20, and the guide wire main body (specifically, a portion of the first tapered portion 52). As illustrated in Fig. 1, the third fixation portion 73 is provided entirely in the circumferential direction between the inner peripheral surface 33 of the third tube 30 and the inner peripheral surface 23 of the second tube 20 and the outer peripheral surface of the first tapered portion 52 and blocks the flow of gas between the gas layer 43 and the gas layer 42.

The fourth fixation portion 74 is a member that is provided at the proximal end portion 22 of the second tube 20 and fixes the proximal end portion 22 of the second tube 20 and the guide wire main body (specifically, the proximal end portion of the large diameter portion 54). As illustrated in Fig. 1, the fourth fixation portion 74 is provided entirely in the circumferential direction between the inner peripheral surface 23 of the second tube 20 and the outer peripheral surface of the large diameter portion 54 and blocks the flow of the gas between the inside and the outside of the plasma guide wire 1 (specifically, the flow of the gas forming the gas layer 42). Furthermore, the fourth fixation portion 74 corresponds to a "proximal end fixation portion". The first fixation portion 71, the second fixation portion 72, the third fixation portion 73, and the fourth fixation portion 74 are collectively and simply referred to as a "fixation portion".

Here, a section in which the guide wire main body is covered with the first tube 10 is referred to as a "first section S1", a section in which the guide wire main body is covered with the second tube 20 is referred to as a "second section S2", and a section in which the guide wire main body is covered with the third tube 30 is referred to as a "third section S3". At this time, in the plasma guide wire 1 according to the present embodiment, the fixation portions are provided in all of the first section S1, the second section S2, and the third section S3. Further, as illustrated in Fig. 1, in the plasma guide wire 1 according to the present embodiment, the proximal end portion of the first section S1 and the distal end portion of the third section S3 are partially overlapped with each other, and the proximal end portion of the third section S3 and the distal end portion of the second section S2 are partially overlapped with each other.

The distal end marker 81 has an insulating property, is colored in an arbitrary color, and functions as a mark indicating the position of the distal end electrode 40. The distal end marker 81 is an annular member provided to surround the outer peripheral surface 14 of the first tube 10 at the distal end portion 11 of the first tube 10.

The core shaft 50 and the distal end electrode 40 may be made of any material having conductivity, for example, chromium-molybdenum steel, nickel-chromium-molybdenum steel, stainless steel such as SUS304, a nickel-titanium alloy, or the like. The distal end electrode 40 may be formed by melting the distal end portion of the core shaft 50 with a laser or the like.

The first tube 10, the second tube 20, the third tube 30, and the distal end marker 81 may be made of any material having an insulating property, for example, copolymers (PFA) of tetrafluoroethylene and perfluoroalkoxy ethylene, polyethylene, polypropylene, polyolefin such as ethylene-propylene copolymers, polyester such as polyethylene terephthalate, polyvinyl chloride, ethylene-vinyl acetate copolymers, crosslinked ethylene-vinyl acetate copolymers, thermoplastic resin such as polyurethane, polyamide elastomer, polyolefin elastomer, silicone rubber, latex rubber, polyetheretherketone, polyetherimide, polyamide-imide, polysulfone, polyimide, and super engineering plastic such as polyethersulfone. The first tube 10, the second tube 20, the third tube 30, and the distal end marker 81 may be made of the same material, or may be made of different materials depending on the performance required for the plasma guide wire 1 (for example, flexibility, torquability, shape maintainability, and the like, of the distal end portion).

The coil fixation portion 70, the first fixation portion 71, the second fixation portion 72, the third fixation portion 73, and the fourth fixation portion 74 may be made of any bonding agent, for example, any bonding agent such as an epoxy-based adhesive.

With the plasma guide wire 1 of Fig. 1, an operator delivers the plasma guide wire 1 to the vicinity of a living tissue (for example, CTO) which is an ablation target, and then outputs high-frequency power from the RF generator 100 in a state where the distal end electrode 40 of the plasma guide wire 1 is positioned in the vicinity of the CTO. Then, due to a potential difference between the distal end electrode 40 of the plasma guide wire 1 and another electrode of another device, streamer corona discharge occurs between the distal end electrode 40 and another electrode. By this streamer corona discharge, the CTO in the vicinity of the distal end electrode 40 of the plasma guide wire 1 may be ablated. In the plasma guide wire 1 according to the present embodiment, since the guide wire main body to which a high-frequency current is applied is fixed to the first tube 10, the second tube 20, and the third tube 30 for providing electrical insulation, excessive care is not required for handling, and safety may be improved, as compared with a case where these tubes are insertable and removable.

As described above, in the plasma guide wire 1 according to the first embodiment, since the guide wire main body (the core shaft 50 and the coil body 60) is covered with the first tube 10, the second tube 20, and the third tube 30 each made of an insulating resin, it is possible to insulate the guide wire main body from the outside and to give insulating performance to the plasma guide wire 1. Furthermore, the gas layer 41, the gas layer 42, and the gas layer 43 filled with gas are formed between the first tube 10, the second tube 20, and the third tube 30 and the guide wire main body, respectively. Here, when air is given as an example of the gas, the volume resistivity of air is higher than the volume resistivity of resin, and thus it may be said that air has a higher insulation performance than resin. In addition, it is known that, for example, sulfur hexafluoride (SF6) gas has better insulating properties than air. Therefore, by providing the plasma guide wire 1 with the gas layers 41, 42, 43 filled with gas having such a high insulation performance, the insulation performance of the plasma guide wire 1 may be improved as compared with a configuration without a gas layer. As a result, the operator may safely perform the plasma ablation treatment using the plasma guide wire 1.

Here, the first tube 10 covering the distal end side of the guide wire main body (the core shaft 50 and the coil body 60) may be formed to have the thin thickness T10 or may be made of a flexible resin material in order to improve flexibility as compared with the second tube 20 covering the proximal end side of the guide wire main body and the third tube 30 covering the intermediate portion of the guide wire main body. In this respect, with the plasma guide wire 1 according to the first embodiment, since the gas layer 41 filled with gas is formed between the first tube 10 and the guide wire main body, even when the insulation performance of the first tube 10 itself is inferior to that of the second tube 20 or the third tube 30, the gas layer 41 may improve the insulation performance of the portion where the first tube 10 is located (in other words, the distal end side portion of the plasma guide wire 1).

Furthermore, in the plasma guide wire 1 according to the first embodiment, since the thicknesses of the gas layers 41, 42, 43 are 1 µm or more, it is possible to secure an insulation distance necessary for suppressing electric leakage, and it is possible to suppress the occurrence of electric leakage and the occurrence of dielectric breakdown due to electric leakage. Further, even when electric leakage occurs, the amount of leakage current may be reduced. When the thicknesses of the gas layers 41, 42, 43 are less than 1 µm, a sufficient insulation distance cannot be secured, and thus electric leakage or an increase in the amount of leakage current may occur during manipulation. In addition, in the plasma guide wire 1 according to the first embodiment, since the thicknesses of the gas layers 41, 42, 43 are 100 µm or less, both the insulation performance and the usability (for example, delivery performance and operability) of the plasma guide wire 1 may be achieved. When the thicknesses of the gas layers 41, 42, 43 are more than 100 µm, the insulation performance may be improved, but the usability (for example, delivery performance and operability) of the plasma guide wire 1 is reduced. For example, when the outer diameters φ10, φ20, φ30 of the first, second, and third tubes 10, 20, 30 are increased in order to make the thicknesses of the gas layers 41, 42, 43 more than 100 µm, the diameter of the plasma guide wire 1 is increased, and the delivery performance of the plasma guide wire 1 to bent portions of blood vessels and thin blood vessels is reduced. In addition, for example, when the outer diameter of the core shaft 50 is reduced in order to make the thicknesses of the gas layers 41, 42, 43 more than 100 µm, the mechanical performance (for example, torquability and support properties) of the plasma guide wire 1 is reduced, and the operability of the plasma guide wire 1 is reduced.

Further, in the plasma guide wire 1 according to the first embodiment, the distal end fixation portion (the first fixation portion 71) is provided at the distal end portion of the first tube 10, the proximal end fixation portion (the fourth fixation portion 74) is provided at the proximal end portion of the second tube 20, and the distal end fixation portion and the proximal end fixation portion block the flow of gas between the inside and the outside of the plasma guide wire 1. Therefore, it is possible to suppress the gas forming the gas layers 41, 42, 43 inside the plasma guide wire 1 from leaking to the outside of the plasma guide wire 1 when the plasma guide wire 1 is grasped or curved during a procedure. As a result, the insulation performance of the plasma guide wire 1 may be further improved.

Further, in the plasma guide wire 1 according to the first embodiment, as illustrated in Fig. 2A, the outer diameter φ50b of the intermediate portion of the guide wire main body (the core shaft 50 and the coil body 60) is smaller than the outer diameter φ50a on the distal end side of the guide wire main body and is smaller than the outer diameter φ50c on the proximal end side of the guide wire main body. Furthermore, as illustrated in Fig. 2B, the outer diameter φ30 of the third tube 30 covering the intermediate portion is smaller than the outer diameter φ10 of the first tube 10 covering the distal end side, and is smaller than the outer diameter φ20 of the second tube 20 covering the proximal end side. Therefore, the outer shape of the plasma guide wire 1 (specifically, the outer shapes of the first, second, and third tubes 10, 20, 30) may be formed along the outer shape of the guide wire main body. As a result, the diameter of the plasma guide wire 1 may be reduced, and the mechanical performance (for example, torquability and support characteristics) of the plasma guide wire 1 may be maintained.

Furthermore, with the plasma guide wire 1 according to the first embodiment, the third tube 30 is provided such that the distal end portion 31 is overlapped with the proximal end portion 12 of the first tube 10 and the proximal end portion 32 is overlapped with the distal end portion 21 of the second tube 20, and the outer peripheral surface 34 of the third tube 30 is joined to the inner peripheral surfaces 13, 23 of the other tubes (the first and second tubes 10, 20) so that the leakage of the gas from the boundary between the third tube 30 and the other tubes (the first and second tubes 10, 20) may be suppressed. In other words, with the plasma guide wire 1 according to the first embodiment, it is possible to suppress the gas forming the gas layers 41, 42, 43 from leaking to the outside from the distal end side joint portion 82 and the proximal end side joint portion 83.

Furthermore, with the plasma guide wire 1 according to the first embodiment, since at least the intermediate portion of the third tube 30 located between the distal end portion 31 and the proximal end portion 32 is not covered with the first tube 10 and the second tube 20 and is exposed to the outside, the diameter of the plasma guide wire 1 in the third section S3 (Fig. 1: the section in which the guide wire main body is covered with the third tube 30) may be reduced, and the plasma guide wire 1 may be flexibly configured.

### <Second Embodiment>

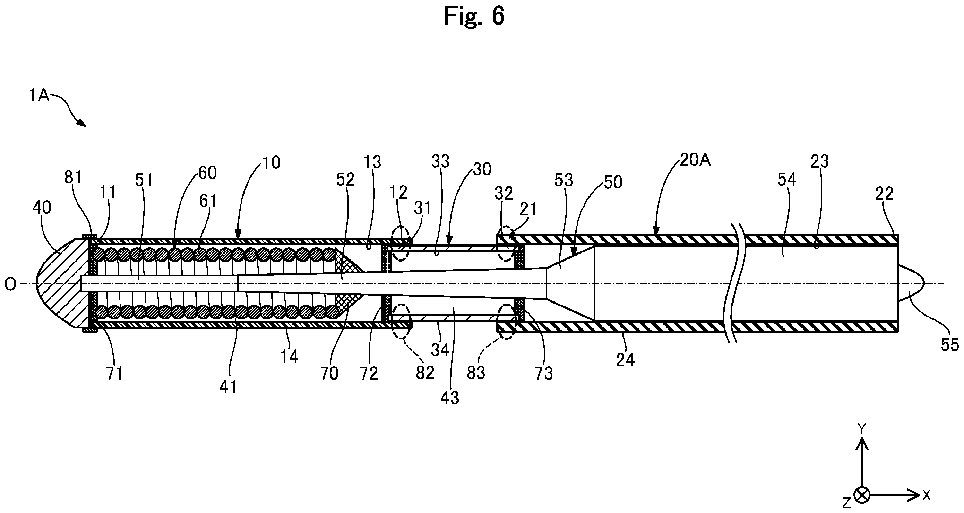

Fig. 6 is an explanatory view exemplifying a sectional configuration of a plasma guide wire 1A according to a second embodiment. The plasma guide wire 1A according to the second embodiment includes a second tube 20A instead of the second tube 20 in the configuration described in the first embodiment, and does not include the gas layer 42 and the fourth fixation portion 74. As illustrated in Fig. 6, in the second tube 20A, the inner peripheral surface 23 of the second tube 20A is in contact with the outer peripheral surface of the large diameter portion 54 of the core shaft 50. Therefore, the gas layer 42 filled with gas is not formed between the second tube 20A and the guide wire main body. In addition, since the second tube 20A and the large diameter portion 54 are provided in contact with each other, the fourth fixation portion 74, which fixes the second tube 20 and the large diameter portion 54 in Fig. 1, is omitted.

As described above, the configuration of the plasma guide wire 1A may be variously changed, and no gas layer may be formed between the second tube 20A and the guide wire main body. Although no gas layer is formed in the second tube 20A in the example of Fig. 6, no gas layer may be formed between the first tube 10 and the guide wire main body (in other words, the gas layer 41 may be omitted), and no gas layer may be formed between the third tube 30 and the guide wire main body (in other words, the gas layer 43 may be omitted). The plasma guide wire 1A according to the second embodiment described above may also achieve the same effects as those of the first embodiment described above.

### <Third Embodiment>

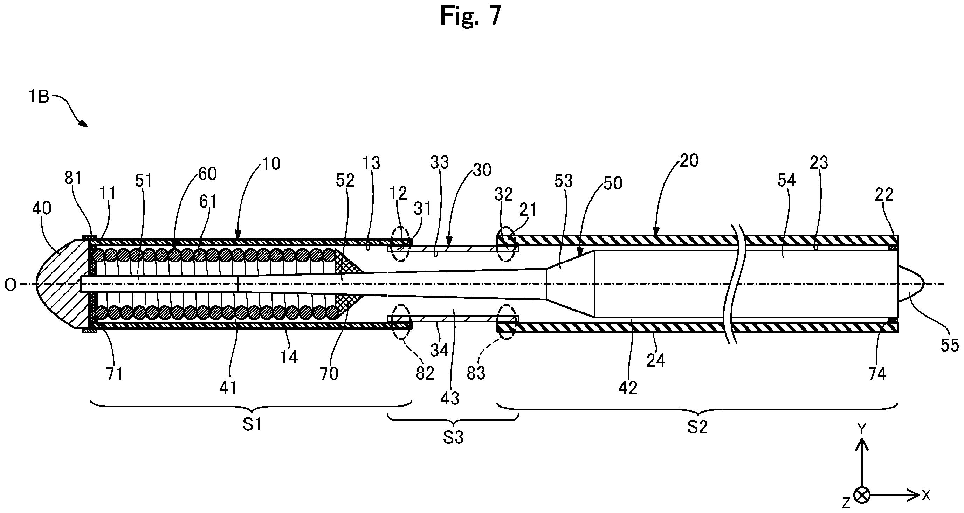

Fig. 7 is an explanatory view exemplifying a sectional configuration of a plasma guide wire 1B according to a third embodiment. The plasma guide wire 1B according to the third embodiment does not include the second fixation portion 72 and the third fixation portion 73 in the configuration described in the first embodiment. In the plasma guide wire 1B illustrated in Fig. 7, the third tube 30 is only joined to the first tube 10 by the distal end side joint portion 82 and is joined to the second tube 20 by the proximal end side joint portion 83, and is not fixed to the core shaft 50. In other words, in the plasma guide wire 1B, no fixation portion is provided in the third section S3.

As described above, the configuration of the plasma guide wire 1B may be variously changed, and no fixation portion may be provided in the third section S3. In addition, in the example of Fig. 7, the case where no fixation portion is provided in the third section S3 by omitting the second fixation portion 72 and the third fixation portion 73 is illustrated. However, the configuration in which no fixation portion is provided in the first section S1 by omitting the first fixation portion 71 and the second fixation portion 72 may be adopted, and the configuration in which no fixation portion is provided in the second section S2 by omitting the third fixation portion 73 and the fourth fixation portion 74 may be adopted. Further, for example, only the second fixation portion 72 may be omitted, only the third fixation portion 73 may be omitted, or only the fourth fixation portion 74 may be omitted. Further, an additional fixation portion which is not described in the first embodiment may be further provided.

With the plasma guide wire 1B according to the third embodiment as described above, since the third tube 30 is joined to the first tube 10 and the second tube 20 by the distal end side joint portion 82 and the proximal end side joint portion 83, the same effects as those in the first embodiment described above may be achieved. Further, with the plasma guide wire 1B according to the third embodiment, since no fixation portion is provided in at least any one of the first section S1, the second section S2, and the third section S3, the flexibility of the plasma guide wire 1 may be improved as compared with the configuration in which the fixation portion is provided in all of the first, second, and third sections S1, S2, S3.

### <Fourth Embodiment>

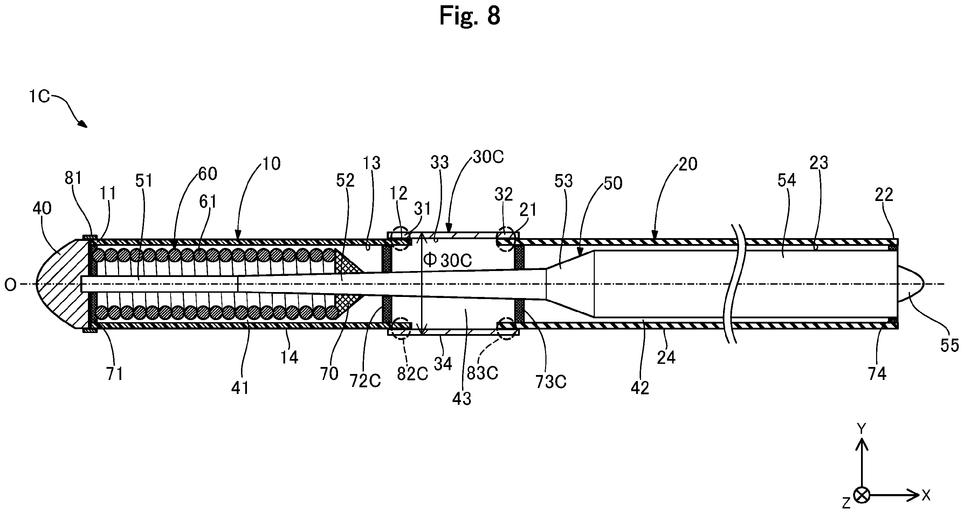

Fig. 8 is an explanatory view exemplifying a sectional configuration of a plasma guide wire 1C according to a fourth embodiment. The plasma guide wire 1C according to the fourth embodiment includes a third tube 30C instead of the third tube 30, a second fixation portion 72C instead of the second fixation portion 72, a third fixation portion 73C instead of the third fixation portion 73, a distal end side joint portion 82C instead of the distal end side joint portion 82, and a proximal end side joint portion 83C instead of the proximal end side joint portion 83 in the configuration described in the first embodiment.

An outer diameter φ30C of the third tube 30C is larger than the outer diameter φ10 of the first tube 10 illustrated in Fig. 2B and is larger than the outer diameter φ20 of the second tube 20 illustrated in Fig. 2B. As illustrated in Fig. 8, the third tube 30C is provided such that the distal end portion 31 of the third tube 30C is overlapped with the proximal end portion 12 of the first tube 10 and the proximal end portion 32 of the third tube 30C is overlapped with the distal end portion 21 of the second tube 20. Therefore, the inner peripheral surface 33 of the distal end portion 31 of the third tube 30C is joined to the outer peripheral surface 14 of the proximal end portion 12 of the first tube 10. Similarly, the inner peripheral surface 33 of the proximal end portion 32 of the third tube 30C is joined to the outer peripheral surface 24 of the distal end portion 21 of the second tube 20. In the configuration of Fig. 8, the entire third tube 30C from the distal end to the proximal end is not covered with the first tube 10 or the second tube 20 and is exposed to the outside. In the configuration of Fig. 8, since the joining relation (inner surface/outer surface) of the third tube 30, the first tube 10, and the second tube 20 is opposite to that according to the first embodiment, the joint portion between the third tube 30 and the first tube 10 is illustrated as a distal end side joint portion 82C, and a joint portion between the third tube 30 and the second tube 20 is illustrated as a proximal end side joint portion 83C.

The second fixation portion 72C is provided at the proximal end portion 12 of the first tube 10 and fixes the proximal end portion 12 of the first tube 10 and the guide wire main body (to be specific, a portion of the first tapered portion 52). The third fixation portion 73C is provided at the distal end portion 21 of the second tube 20 and fixes the distal end portion 21 of the second tube 20 and the guide wire main body (to be specific, a portion of the first tapered portion 52). The second fixation portion 72C and the third fixation portion 73C may be configured to also fix the third tube 30 as in the first embodiment.

As described above, the configuration of the plasma guide wire 1C may be variously changed, and the outer diameter φ30C of the third tube 30C, the outer diameter φ10 of the first tube 10, and the outer diameter φ20 of the second tube 20 may be arbitrarily changed. For example, as illustrated in Fig. 8, the outer diameter φ30C of the third tube 30C may be formed to be the largest so that the third tube 30C covers the proximal end portion 12 of the first tube 10 and the distal end portion 21 of the second tube 20. Alternatively, the outer diameters of the first, second, and third tubes 10, 20, 30C may be gradually decreased from the proximal end side toward the distal end side (in other words, φ20 > φ30C > φ10), and the outer diameter of the plasma guide wire 1C may be gradually decreased from the proximal end side toward the distal end side. The plasma guide wire 1C according to the fourth embodiment described above may also achieve the same effects as those of the first embodiment described above.

### <Fifth Embodiment>

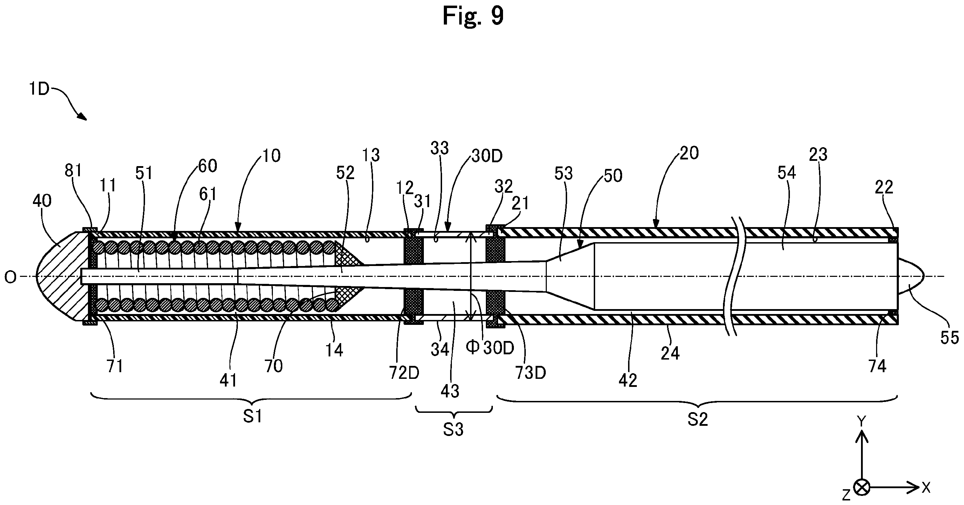

Fig. 9 is an explanatory view exemplifying a sectional configuration of a plasma guide wire 1D according to a fifth embodiment. The plasma guide wire 1D according to the fifth embodiment includes a third tube 30D instead of the third tube 30, a second fixation portion 72D instead of the second fixation portion 72, and a third fixation portion 73D instead of the third fixation portion 73 in the configuration described in the first embodiment. Further, the plasma guide wire 1D according to the fifth embodiment does not include the distal end side joint portion 82 and the proximal end side joint portion 83 described in the first embodiment.

An outer diameter φ30D of the third tube 30D is equal to the outer diameter φ10 of the first tube 10 illustrated in Fig. 2B and is equal to the outer diameter φ20 of the second tube 20 illustrated in Fig. 2B. Here, "the same" and "equal" are not limited to a case of a strict match and have a meaning allowing differences due to manufacturing errors, or the like. The distal end portion 31 of the third tube 30D is located closer to the proximal end side than the proximal end portion 12 of the first tube 10, and the third tube 30D and the first tube 10 are not overlapped with each other. Similarly, the proximal end portion 32 of the third tube 30D is located closer to the distal end side than the distal end portion 21 of the second tube 20, and the third tube 30D and the second tube 20 are not overlapped with each other. Further, the distal end portion 31 of the third tube 30D is not joined to the first tube 10, and the proximal end portion 32 of the third tube 30D is not joined to the second tube 20.

The second fixation portion 72D fills the distal end portion 31 of the third tube 30D, the proximal end portion 12 of the first tube 10, and a clearance between the third tube 30D and the first tube 10. As illustrated in Fig. 9, since the second fixation portion 72D is formed to rise on the outer peripheral surfaces of the third tube 30D and the first tube 10, leakage of the gas from the second fixation portion 72D may be suppressed. The third fixation portion 73D fills the proximal end portion 32 of the third tube 30D, the distal end portion 21 of the second tube 20, and a clearance between the third tube 30D and the second tube 20. Since the third fixation portion 73D is formed to rise on the outer peripheral surfaces of the third tube 30D and the second tube 20, the leakage of the gas from the third fixation portion 73D may be suppressed. Further, as illustrated in Fig. 9, in the plasma guide wire 1D, the first section S1 in which the guide wire main body is covered with the first tube 10, the second section S2 in which the guide wire main body is covered with the second tube 20, and the third section S3 in which the guide wire main body is covered with the third tube 30D are not overlapped with each other.

As described above, the configuration of the plasma guide wire 1D may be variously changed, and the third tube 30D may not be overlapped with the first tube 10 or the second tube 20. The plasma guide wire 1D according to the fifth embodiment described above may also achieve the same effects as those of the first embodiment described above. Further, with the plasma guide wire 1D according to the fifth embodiment, the outer diameter of the plasma guide wire 1D may be formed uniformly.

### <Sixth Embodiment>

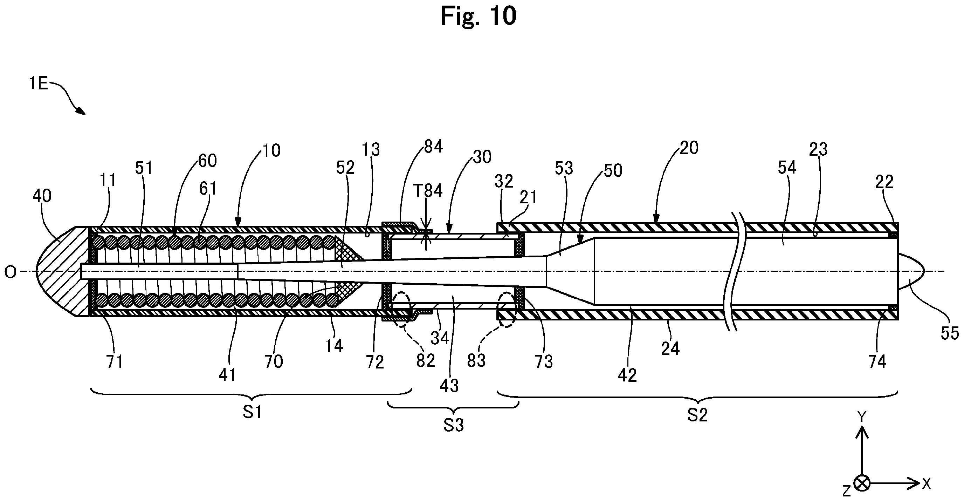

Fig. 10 is an explanatory view exemplifying a sectional configuration of a plasma guide wire 1E according to a sixth embodiment. The plasma guide wire 1E according to the sixth embodiment further includes a shrink tube 84 and does not include the distal end marker 81 in the configuration described in the first embodiment.

The shrink tube 84 is a heat-shrinkable tube that covers the joint portion (the distal end side joint portion 82) between the first tube 10 and the third tube 30. A thickness T84 of the shrink tube 84 is thinner than the thickness T 10 of the first tube 10 illustrated in Fig. 2B, is thinner than the thickness T20 of the second tube 20, and is thinner than the thickness T30 of the third tube 30. The shrink tube 84 has a stepped annular shape in which the distal end side has an inner diameter along the outer diameter φ10 of the first tube 10 and the proximal end side has an inner diameter along the outer diameter φ30 of the third tube 30. The shrink tube 84 is formed into the shape illustrated in Fig. 10 by being placed over the outside of the joint portion (the distal end side joint portion 82) between the first tube 10 and the third tube 30 and then being heated.

As described above, the configuration of the plasma guide wire 1E may be variously changed, and another configuration not described in the first embodiment, such as the shrink tube 84, may be further provided, and the configuration described in the first embodiment, such as the distal end marker 81, may be omitted. The plasma guide wire 1E according to the sixth embodiment described above may also achieve the same effects as those of the first embodiment described above.

Further, with the plasma guide wire 1E according to the sixth embodiment, the joint portion (the distal end side joint portion 82) between the first tube 10 and the third tube 30 is covered with the shrink tube 84. For this reason, it is possible to suppress separation of the first tube 10 and the third tube 30 due to peeling occurring in the distal end side joint portion 82, and it is possible to suppress leakage of the gas from the location where peeling has occurred. In addition, since the thickness T84 of the shrink tube 84 is thinner than the thicknesses T10, T20, T30, the diameter of the plasma guide wire 1 may be kept small as compared with the case where the first, second, and third tubes 10, 20, 30 having an insulating property are stacked. Furthermore, when the plasma guide wire 1 is used in combination with a catheter including other electrodes, the distal end portion of the plasma guide wire 1 (in particular, the first section S1 and the second section S2) protrudes from a distal end opening of the catheter during use. Since the distal end side in particular (the first section S1 and the second section S2 in particular) of the plasma guide wire 1 is configured to be flexible, the distal end opening of the catheter and the joint portion (the distal end side joint portion 82) between the first tube 10 and the third tube 30 may scrape against each other when the plasma guide wire 1 is caused to protrude from the distal end opening of the catheter. In this regard, with the plasma guide wire 1E according to the sixth embodiment, the shrink tube 84 may achieve a structure resistant to friction.

### <Seventh Embodiment>

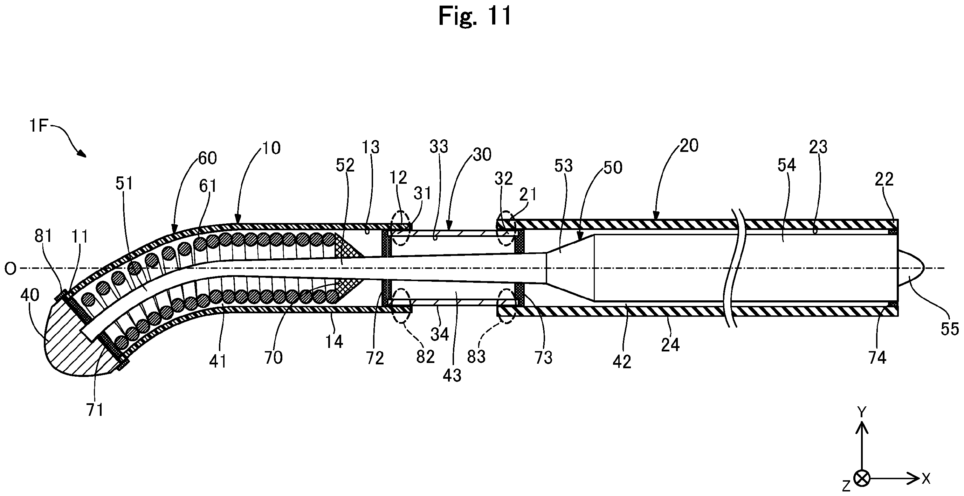

Fig. 11 is an explanatory view exemplifying a sectional configuration of a plasma guide wire 1F according to a seventh embodiment. In the plasma guide wire 1F according to the seventh embodiment, the distal end side of the plasma guide wire 1F is pre-shaped in the configuration described in the first embodiment. In other words, a curved shape is applied to the distal end side of the plasma guide wire 1F. In the example illustrated in Fig. 11, in the plasma guide wire 1, the first tube 10 and the guide wire main body covered with the first tube 10 are curved. As described above, even when the distal end side of the plasma guide wire 1 is pre-shaped, the gas layer 41 is present entirely in the circumferential direction between the first tube 10 and the guide wire main body and entirely in the longitudinal direction from the distal end portion to the proximal end portion of the first tube 10 as in the first embodiment.

As described above, the configuration of the plasma guide wire 1F may be variously changed, and the distal end side of the plasma guide wire 1 may be pre-shaped. In the example of Fig. 11, a case where the first tube 10 and the guide wire main body covered with the first tube 10 are curved is illustrated, but in addition to the first tube 10, the third tube 30 and the guide wire main body covered with the third tube 30 may also be curved. Even in this case, the gas layer 43 is present entirely in the circumferential direction between the third tube 30 and the guide wire main body and entirely in the longitudinal direction from the distal end portion to the proximal end portion of the third tube 30, as in the first embodiment.

The plasma guide wire 1F according to the seventh embodiment described above may also achieve the same effects as those of the first embodiment described above. Further, with the plasma guide wire 1F according to the seventh embodiment, since the distal end side is pre-shaped, the vascular selectivity by the plasma guide wire 1F may be improved. Further, since the distal end side is pre-shaped, the angle formed by the distal end portion of the plasma guide wire 1F and the living tissue may be increased. As a result, the depth of the hole formed in the living tissue may be further increased by the ablation using the plasma guide wire 1F so that the effect of the ablation may be easily obtained.

### <Modification of Present Embodiment>

The disclosed embodiments are not limited to the above-described embodiments and may be implemented in various modes without departing from the gist thereof, and for example, the following modifications are also possible.

### [Modification 1]

In the first to seventh embodiments, examples of the configurations of the plasma guide wires 1, 1A to 1F have been described. However, the configurations of the plasma guide wires 1, 1A to 1F may be variously changed. For example, the first tube 10, the second tube 20, and the third tube 30 may be integrally formed. For example, in order to reduce the diameter of the plasma guide wire 1, the thickness of each of the gas layers 41, 42, 43 may be less than 1 µm. For example, for the purpose of further improving the insulation performance of the plasma guide wire 1, the thicknesses of the gas layers 41, 42, 43 may be more than 100 µm. For example, a configuration is possible in which the first fixation portion 71 does not fix the core shaft 50. In this case, the first fixation portion 71 fixes the distal end portion 11 of the first tube 10 and the coil body 60 (guide wire main body) and may allow the flow of gas between the inside and the outside of the plasma guide wire 1.

For example, the core shaft 50 forming the guide wire main body is not limited to the above-described shape, and may have an arbitrary shape. For example, at least a part of the small diameter portion 51, the first tapered portion 52, the second tapered portion 53, the large diameter portion 54, and the proximal end portion 55 exemplified in the above embodiments may be omitted. For example, the guide wire main body may include additional configurations not described above. For example, an inner coil body may be provided inside the coil body 60.

### [Modification 2]

The configurations of the plasma guide wires 1, 1A to 1F according to the first to seventh embodiments and the configurations of the plasma guide wires 1, 1A to 1F according to the modification 1 may be combined as appropriate. For example, in the plasma guide wires 1, 1A to 1E described in the second to sixth embodiments, the configuration in which the distal end side described in the seventh embodiment is pre-shaped may be adopted. For example, in the plasma guide wires 1, 1A to 1D described in the second to fifth embodiments, the shrink tube 84 described in the sixth embodiment may be provided. For example, in the plasma guide wires 1A and B described in the second and third embodiments, the third tube 30C described in the fourth embodiment may be provided. For example, in the plasma guide wire 1A described in the second embodiment, a configuration in which the second fixation portion 72 and the third fixation portion 73 described in the third embodiment are omitted may be adopted.

Although the present mode has been described above based on the embodiments and the modifications, the embodiment of the above-described mode is intended to facilitate understanding of the present mode and does not limit the present mode. The present mode may be modified and improved without departing from the gist and the scope of the claims, and the present mode includes equivalents thereof. In addition, if the technical features are not described as essential in the present specification, the technical features may be appropriately deleted.

### DESCRIPTION OF REFERENCE NUMERALS

1, 1A to 1F Plasma guide wire
10 First tube
11 Distal end portion
12 Proximal end portion
13 Inner peripheral surface
14 Outer peripheral surface
20, 20a Second tube
21 Distal end portion
22 Proximal end portion
23 Inner peripheral surface
24 Outer peripheral surface
30, 30c, 30d Third tube
31 Distal end portion
32 Proximal end portion
33 Inner peripheral surface
34 Outer peripheral surface
40 Distal end electrode
41, 42, 43 Gas layer
50 Core shaft
51 Small diameter portion
52 First tapered portion
53 Second tapered portion
54 Large diameter portion
55 Proximal end portion
60 Coil body
61 Wire
70 Coil fixation portion
71 First fixation portion
72, 72c, 72d Second fixation portion
73, 73c, 73d Third fixation portion
74 Fourth fixation portion
81 Distal end marker
82, 82c Distal end side joint portion
83, 83c Proximal end side joint portion
84 Shrink tube
100 RF generator
110 First terminal
111 First cable
120 Second terminal
121 Second cable

## Claims

1. A plasma guide wire comprising:
a guide wire main body including a core shaft and a coil body provided to surround a portion of the core shaft on a distal end side;
a hollow cylindrical first tube that is made of an insulating resin and covers a distal end side of the guide wire main body;
a hollow cylindrical second tube that is made of an insulating resin and covers a proximal end side of the guide wire main body; and
a hollow cylindrical third tube that is made of an insulating resin, covers an intermediate portion of the guide wire main body located between the distal end side and the proximal end side, has a distal end portion joined to a proximal end portion of the first tube, and has a proximal end portion joined to a distal end portion of the second tube,
wherein at least any one of the first tube, the second tube, and the third tube forms a gas layer filled with gas, in conjunction with the guide wire main body.

2. The plasma guide wire according to claim 1, wherein the gas layer filled with the gas is formed between the first tube and the guide wire main body.

3. The plasma guide wire according to claim 1 or 2, wherein a thickness of the gas layer is 1 µm or more and 100 µm or less.

4. The plasma guide wire according to any one of claims 1 to 3, further comprising a fixation portion that fixes any of the first tube, the second tube, and the third tube to the guide wire main body,
wherein the fixation portion is not provided in at least any one of a first section in which the guide wire main body is covered with the first tube, a second section in which the guide wire main body is covered with the second tube, and a third section in which the guide wire main body is covered with the third tube.

5. The plasma guide wire according to any one of claims 1 to 3, further comprising:
a distal end fixation portion that is provided at a distal end portion of the first tube and fixes the first tube and the guide wire main body; and
a proximal end fixation portion that is provided at a proximal end portion of the second tube and fixes the second tube and the guide wire main body,
wherein the distal end fixation portion and the proximal end fixation portion block flow of the gas between inside and outside of the plasma guide wire.

6. The plasma guide wire according to any one of claims 1 to 5,
wherein an outer diameter of the intermediate portion of the guide wire main body is smaller than an outer diameter of the guide wire main body on the distal end side and is smaller than an outer diameter of the guide wire main body on the proximal end side, and
wherein an outer diameter of the third tube covering the intermediate portion is smaller than an outer diameter of the first tube covering the distal end side and is smaller than an outer diameter of the second tube covering the proximal end side.

7. The plasma guide wire according to claim 6,
wherein the third tube is provided such that the distal end portion of the third tube is overlapped with the proximal end portion of the first tube and the proximal end portion of the third tube is overlapped with the distal end portion of the second tube,
wherein an outer peripheral surface of the distal end portion of the third tube is joined to an inner peripheral surface of the proximal end portion of the first tube, and
wherein an outer peripheral surface of the proximal end portion of the third tube is joined to an inner peripheral surface of the distal end portion of the second tube.

8. The plasma guide wire according to any one of claims 1 to 7, wherein an intermediate portion of the third tube located at least between the distal end portion and the proximal end portion is not covered with the first tube and the second tube and is exposed to outside.
